# EUROPEAN PATENT APPLICATION

(11) **EP 4 008 773 A1**
(43) Date of publication of application: **08.06.2022**
(21) Application number: 20851059.4
(22) Date of filing: 05.06.2020
(51) Int. Cl.: C12N 5/00

(54) **POULTRY EGG CULTURE MEDIUM**

(30) Priority: 03.08.2019 US 201962882479 P
(71) Applicant: Shenzhen Mytang Biotechnology Co., Ltd., Shenzhen, Guangdong 518000 (CN)
(72) Inventor: TANG, Lingfeng, Shenzhen, Guangdong 518000 (CN); WU, Yihua, Shenzhen, Guangdong 518000 (CN); WANG, Yue, Shenzhen, Guangdong 518000 (CN)
(74) Representative: Kolster Oy Ab
(86) International application number: PCT/CN2020/094721
(87) International publication number: WO 2021/022892

(57) **Abstract**

Disclosed is a poultry egg-based culture medium related to the field of biotechnology, in particular a culture medium that can be used for cultivating mammalian cells, the culture medium containing a poultry egg content and a diluent. Also related is a preparation and use of the culture medium, and a method for using the culture medium for cultivating mammalian cells.

## Description

### BACKGROUND

### Technical Field

The present invention relates to the field of biotechnology, in particular to a poultry egg-based culture medium that can be used for cultivating mammalian cells, preparation therefor and use thereof.

### Description of Related Art

Mammalian cells play an important role in cell therapy, drug development, and production of artificial meat based on cell culture. The existing mammalian cell culture technique is very costly, mainly because the cell culture medium is very expensive. Every 500 mL of common Dulbecco's Modified Eagle Medium (DMEM), or Roswell Park Memorial Institute (RPMI) medium costs nearly 100 yuan. At the same time, fetal bovine serum (FBS) is commonly required for cell culture. The price of FBS is extremely high, usually costing several thousands of yuan for every 500 mL. To get 50 g of mammalian cells by cell culture, the cost of culture media alone would reach about 500,000 yuan. The high cost of culture media greatly limits the large-scale production of mammalian cells. Therefore, there is a need in the art for a culture medium that is low-cost, has a wide range of raw materials for preparing the culture medium, and can be produced on a large scale for cultivating mammalian cells.

### BRIEF SUMMARY OF THE INVENTION

Poultry eggs can develop into complete individuals, which themselves contain all the nutrients needed for the growth of avian cells. At the same time, poultry eggs contain high concentrations of lysozyme, which can inhibit bacterial infections. The present inventors creatively discovered that poultry eggs can be used to prepare a basic medium required for cultivating mammalian cells, thereby greatly reducing the cost of cultivating mammalian cells.

Therefore, in one aspect, the present application provides a culture medium for cultivating mammalian cells, and the culture medium comprises a poultry egg content and a diluent, wherein the poultry egg content has a volume percentage ranging from 0.1% to 90% in the culture medium.

In the present invention, the poultry egg content refers to an egg yolk and/or egg white of the poultry egg, and the poultry egg includes, but is not limited to, hen egg, duck egg, goose egg, ostrich egg, quail egg, or any combination thereof. The poultry egg content may be unfrozen or thawed after being frozen.

In some embodiments, the diluent is a buffer solution, including but not limited to phosphate-buffered saline (PBS), Ringer's solution, Hank's Balanced Salt Solution, or (4-(2-hydroxyethyl)-1-piperazineethanesulfonic acid) (HEPES) solution.

In some embodiments, the culture medium further comprises an antibiotic (for example, ampicillin-streptomycin, or a mixed solution of penicillin-streptomycin and amphotericin B); a pH indicator, preferably phenol red; an energy source, which is usually a carbohydrate compound, preferably glucose; an amino acid; a vitamin and/or another organic compound required at a low concentration; a trace element; an inorganic salt (such as sodium bicarbonate); or any combination thereof. It should be pointed out that since the antibiotic, pH indicator, energy source amino acid, vitamin or the organic compound is added in a small amount compared with the poultry egg content and the diluent, the volume of the substances may be negligible when preparing the culture medium.

In some embodiments, the streptomycin has a concentration of 10 to 1000 µg/mL, the penicillin has a concentration of 10 to 1000 U/mL, the chloramphenicol has a concentration of 0.01 to 1 mg/mL, and the amphotericin B has a concentration of 0.025 to 2.5 ug/mL in the culture medium.

In some embodiments, the phenol red contained in the culture medium has a concentration of 1.5 to 50 mg/L.

In some embodiments, the inorganic salt contained in the culture medium has a concentration of 0.01 to 0.25 mol/L.

In some embodiments, the glucose contained in the culture medium has a concentration of 0.1 to 5 g/L.

In some embodiments, the vitamin contained in the culture medium has a concentration of 3 to 300 mg/L.

In some embodiments, the poultry egg content is egg yolk and egg white. In some embodiments, the poultry egg content is egg yolk. In some embodiments, the poultry egg content is egg white. In some embodiments, the egg yolk and egg white are derived from the same type of poultry egg, for example, both are derived from hen egg, both are derived from duck egg, or both are derived from quail egg. In some embodiments, the egg yolk and egg white are derived from different types of poultry eggs. In some embodiments, the ratio of egg yolk to egg white is 99:1 to 1:99.

In some embodiments, the culture medium of the present invention does not contain a FBS. In some embodiments, the culture medium of the present invention may contain a certain amount of FBS, for example, contain no more than 5% (by volume percentage) of FBS.

In one aspect, the present application provides use of the culture medium according to any one of the above items in cultivating mammalian cells.

In some embodiments, the cultivating is adherent cultivating or suspension cultivating.

In some embodiments, the culture medium of the present invention is mixed with another conventional culture medium that can be used for cultivating mammalian cells. For example, the culture medium can be used in combination with another culture medium such as RPMI-1640, DMEM, MEM, M199, F-10, F-12, DMEM/F-12 1:1 or McCoy's 5A.

The culture medium of the present invention can be used for cultivating a mammalian cell, wherein the mammal can be selected from the group consisting of bovine, equine, swine, canine, feline, rodent (e.g., rat or mouse) or primate (e.g., human being).

In some embodiments, the mammalian cell is derived from connective tissue, muscle tissue, nerve tissue, or epithelial tissue. In some embodiments, the mammalian cell is derived from brain, heart, bladder, ovary, pancreas, breast, liver, lung, stomach, kidney, intestine, skin, bone, muscle, esophagus, trachea, male reproductive system (e.g., spermary (testis), epididymis, spermaduct, accessory sexual gland, penis), female reproductive system (e.g., ovary, oviduct, uterus, vagina, external genitalia), lymph, nerve or thyroid.

The culture medium of the present invention can be used for cultivating mammalian non-tumor cells, and can also be used for cultivating tumor cells.

In some embodiments, the mammalian cell is a tumor cell, such as a brain tumor cell, lung cancer cell, squamous cell carcinoma cell, bladder cancer cell, gastric cancer cell, ovarian cancer cell, peritoneal cancer cell, pancreatic cancer cell, breast cancer cell, head and neck cancer cell, cervical cancer cell, endometrial cancer cell, rectal cancer cell, liver cancer cell, kidney cancer cell, esophageal adenocarcinoma cell, esophageal squamous cell cancer cell, prostate cancer cell, female reproductive tract cancer cell, in-situ cancer cell, lymphoma cell, neurofibroma cell, thyroid cancer cell, bone cancer cell, skin cancer cell, brain cancer cell, colon cancer cell, testicular cancer cell, gastrointestinal stromal tumor cell, prostate tumor cell, mast cell tumor cell, multiple myeloma cell, melanoma cell, glioma cell or sarcoma cell.

In some embodiments, the mammalian cell is selected from the group consisting of: human embryonic kidney cell, human T lymphocyte, human cervical cancer cell, mouse breast cancer cell, mouse colon cancer cell, mouse spermatocyte, rat islet cell tumor cell, mouse melanoma cell.

In some embodiments, the mammalian cell is selected from the following cell lines: 293A, 293T, H9, HeLa, 4T1, CT26, GC2-spd, Ins-1 and B16.

In one aspect, the present application further provides a method for preparing the culture medium of the present invention, the method comprising the following steps:
Step 1: obtaining a poultry egg content in an aseptic condition;
Step 2: diluting the poultry egg content with a diluent.

Optionally, the method further comprises: adding an antibiotic, phenol red, glucose, vitamin, inorganic salt, FBS or any combination thereof to the poultry egg content.

In one aspect, the present application further provides a method for cultivating mammalian cells, the method comprising using the culture medium of the present invention.

In some embodiments, the cultivating is adherent cultivating, suspension cultivating or immobilization cultivating.

In some embodiments, the method comprises: mixing the culture medium of the present invention with another culture medium that can be used for cultivating mammalian cells, including but not limited to mixing with RPMI-1640, DMEM, MEM, M199, F-10, F-12, DMEM/F-12 1:1 or McCoy's 5A medium.

The present invention provides a culture medium based on poultry eggs, which can be used without addition of FBS, so that the cost of cultivating mammalian cells can be greatly reduced. The culture medium of the present invention can meet the requirements for growth and proliferation of various mammalian cells, thereby partially or even completely replacing the culture medium containing FBS. The culture medium of the present invention can be used in cell therapy, drug development, production of artificial meat based on cell culture, and in other fields that require a large amount of mammalian cells.

### DETAILED DESCRIPTION OF THE INVENTION

The present invention will be further described in detail below in conjunction with specific embodiments. However, it should be pointed out that the following examples are only for illustrating the present invention, not for limiting the scope of the present invention. The experimental methods in the following examples, unless otherwise specified, were all conventional methods. The materials, reagents, instruments, etc. used in the following examples were obtained from commercial sources unless otherwise specified. For the quantitative experiments in the following examples, each experiment was repeated three times, and the averages of results were taken as final results.

### Example 1: Culture of different cell lines in hen egg-based culture medium

(1) Preparation of culture medium without antibiotics and glucose: fresh hen eggs were taken, their eggshells were opened in aseptic manipulation, their egg white and egg yolk were taken out and weighed, the volume X thereof was calculated using density of 1 g/mL, phenol red (final concentration: 0.0159 g/L) and 0.15M of NaHCO₃ (2.933X) were added, and the final volume was adjusted to 10X (diluted 10 times) with PBS. The prepared culture medium was stored at 4°C for no more than one week.
(2) Preparation of culture medium with antibiotics and glucose: fresh hen eggs were taken, their eggshells were opened in aseptic manipulation, their egg white and egg yolk were taken out and weighed, the volume X thereof was calculated using density of 1 g/mL, phenol red (final concentration: 0.0159 g/L), 0.15M of NaHCO₃ (2.933X), glucose (final concentration: 4.5g/L), ampicillin-streptomycin (final concentrations: 100 units/mL and 100 µg/mL, respectively) were added, and the final volume was adjusted with PBS to 10X (diluted 10 times). The prepared culture medium was stored at 4°C for no more than one week.
(3) The culture medium prepared in step (1) or (2) was used to directly cultivate cells in conventional conditions (37°C, 5% CO₂). For adherent culture, after trypsin digestion, cells were cultivated in a conventional medium (DMEM or RPMI medium, both containing 10% FBS and antibiotics) for 4 to 6 hours to make them adherent, then the DMEM or RPMI medium was then replaced with the poultry egg-based medium. For suspension culture, cells were directly cultivated with the poultry egg-based culture medium. The culture medium was replaced every 1 to 3 days.
(4) A conventional culture medium (DMEM or RPMI medium, both containing 10% FBS and antibiotics) and a serum-free medium (DMEM or RPMI medium, both containing antibiotics only) were used as controls.

The inventors of the present invention compared the cell proliferation folds of different cell lines cultivated in the hen egg-based culture media of the present invention and the conventional culture media (with antibiotics added) for 6 days (a result less than 1 indicates a decrease in the number of cells). The results are shown in Table 1.

**Table 1**

| Cell line | Cell type | Species | Conventional culture medium (containing 10% FBS) | Conventional culture medium (not containing FBS) | Hen egg-based culture medium (containing antibiotics and glucose) | Hen egg-based culture medium (not containing antibiotics or glucose) |
|---|---|---|---|---|---|---|
| 293A Human embryonic kidney cell | Non-tumor adherent cell | Human | 12.40 | 5.00 | 8.33 | 7.50 |
| 293 T Human embryonic kidney cell | Non-tumor adherent cell | Human | 6.80 | 2.00 | 4.20 | 5.40 |
| H9 Human T lymphocyte line | Non-tumor suspension cell | Human | 7.21 | 1.80 | 3.56 | 2.43 |
| HeLa Human cervical cancer cell | Tumor adherent cell | Human | 9.61 | 2.84 | 5.00 | 5.38 |
| 4T1 Mouse breast cancer cell | Tumor adherent cell | Mouse | 5.46 | 0.36 | 3.00 | 3.62 |
| CT26 Mouse colon cancer cell | Tumor adherent cell | Mouse | 18.96 | 0.08 | 1.70 | 2.88 |
| GC2-spd Mouse spermatocyte | Normal adherent cell | Mouse | 12.00 | 6.00 | 8.00 | 5.60 |
| Ins-1 Rat islet cell tumor cell | Tumor adherent cell | Rat | 10.40 | 2.80 | 6.40 | 6.80 |

It may be seen from Table 1 that the hen egg-based culture medium could meet the requirements for growth and proliferation of a variety of mammalian cells and could be used to replace a conventional medium.

Example 2: Culture of different cell lines in duck egg-based medium With reference to the method of Example 1, duck egg-based culture medium without antibiotics and glucose was prepared on the basis of fresh duck eggs and was used in cell culture.

Table 2 shows the cell proliferation folds of different cell lines after being cultivated for 6 days in the duck egg-based culture medium.

**Table 2**

| Cell line | Cell type | Species | Duck egg-based culture medium (not containing antibiotics or glucose) |
|---|---|---|---|
| 293A Human embryonic kidney cell | Non-tumor adherent cell | Human | 6.27 |
| HeLa Human cervical cancer cell | Tumor adherent cell | Human | 3.50 |
| GC2 Mouse spermatocyte | Normal adherent cell | Mouse | 6.62 |
| CT26 Mouse colon cancer cell | Tumor adherent cell | Mouse | 3.42 |

It may be seen from Table 2 that the culture medium based on duck eggs could meet the growth and proliferation requirements of a variety of mammalian cells and could be used to replace a conventional media.

### Example 3: Culture of different cell lines in medium prepared based on frozen-thawed poultry eggs

With reference to the method of Example 1, frozen hen eggs or duck eggs (frozen at -20°C for 1 month) were thawed to prepare a medium without antibiotics and glucose, and a variety of cells were cultivated.

Table 3 shows the cell proliferation folds of different cell lines after being cultivated for 6 days in a medium prepared with thawed eggs or duck eggs.

**Table 3**

| Cell line | Cell type | Species | Hen egg-based culture medium (frozen) | Duck egg-based culture medium (frozen) |
|---|---|---|---|---|
| 293A Human embryonic kidney cell | Non-tumor adherent cell | Human | 8.60 | 6.10 |
| HeLa Human cervical cancer cell | Tumor adherent cell | Human | 4.52 | 4.21 |
| GC2 Mouse spermatocyte | Normal adherent cell | Mouse | 6.53 | 2.13 |
| CT26 Mouse colon cancer cell | Tumor adherent cell | Mouse | 5.46 | 3.30 |

It may be seen from Table 3 that the culture medium prepared based on frozen-thawed poultry eggs could meet the requirements for growth and proliferation of a variety of mammalian cells.

### Example 4: Influences of egg white-egg yolk ratio and dilution ratio on the effect of cultivating 293A cells with egg-based culture medium

With reference to the method of Example 1, a fresh hen egg-based culture medium without antibiotics and glucose was prepared, and the egg white-egg yolk ratio and the dilution ratio were changed, and then cells were cultivated. The results are shown in Table 4.

**Table 4**

| Egg white-egg yolk ratio | Dilution ratio | Cell proliferation folds |
|---|---|---|
| Normal (64.7:35.3) | 1:20 | 10.15 |
| Normal (64.7:35.3) | 1:50 | 5.72 |
| Normal (64.7:35.3) | 1:100 | 4.43 |
| 0:100 | 1:10 | 2.57 |
| 1:99 | 1:10 | 4.29 |
| 2:98 | 1:10 | 7.14 |
| 5:95 | 1:10 | 7.14 |
| 10:90 | 1:10 | 4.26 |
| 25:75 | 1:10 | 6.40 |
| 50:50 | 1:10 | 4:38 |
| 75:25 | 1:10 | 2.76 |
| 90:10 | 1:10 | 1.80 |

| | | |
|---|---|---|
| Note: Dilution was performed using phosphate buffer. | | |

### Example 5: Influences of other factors on the effect of cultivating 293 cells with egg-based culture medium

With reference to the method of Example 1, a fresh hen egg-based culture medium was prepared, and other factors were changed, and then cells were cultivated. The results are shown in Table 5.

**Table 5**

| Purpose | Buffer/solution | Other substance added | Cell proliferation folds |
|---|---|---|---|
| Adding glucose | PBS | Glucose, 2g/L | 17.15 |
| Adding regular medium | PBS | 1% DMEM^{∗} | 2.00 |
| Adding regular medium | PBS | 10%DMEM^{∗} | 5.43 |
| Adding regular medium | PBS | 25%DMEM^{∗} | 7.71 |
| Adding FBS | PBS | 1%FBS | 4.14 |
| Different buffer | Earle's buffer | None | 6.50 |
| Different buffer | Ringer's solution | 1X PSA^{∗∗} | 4.00 |
| Different buffer | Hank's Balanced Salt Solution | 1X PSA^{∗∗} | 8.71 |

| | | | |
|---|---|---|---|
| Note: ^{∗}: DMEM medium was a high-glucose DMEM medium supplemented with 10% FBS and antibiotics (1XPSA); ^{∗∗}: 1X PSA: penicillin-streptomycin-amphotericin B mixed solution: penicillin 100U/mL, chloramphenicol 0.1 mg/mL, amphotericin B 0.25ug/mL. | | | |

The above descriptions are only the preferred examples of the present invention and are not intended to limit the present invention. Any modification, equivalent replacement, improvement, and so on made within the spirit and principle of the present invention shall fall within the protection scope of the present invention.

## Claims

1. A culture medium for cultivating mammalian cells, the culture medium comprising:
a poultry egg content; and
a diluent;
wherein the poultry egg content has a volume percentage ranging from 0.1% to 99% in the culture medium.

2. The culture medium according to claim 1, wherein the culture medium has one or more of the following characteristics:
(1) the poultry egg content is egg yolk and/or egg white of a poultry egg;
(2) the poultry egg is selected from the group consisting of hen egg, duck egg, goose egg, ostrich egg, quail egg, other poultry egg, or any combination thereof;
(3) the poultry egg is unfrozen, or thawed after being frozen;
(4) the diluent is phosphate-buffered saline, Ringer's solution, Hank's Balanced Salt Solution or HEPES buffer;
(5) the culture medium does not contain or contains an antibiotic (e.g., ampicillin-streptomycin, or a mixed solution of penicillin, streptomycin and amphotericin B); a pH indicator, preferably phenol red; an energy source, such as a carbohydrate, preferably, glucose; an amino acid; a vitamin; and/or another organic compound required in a low concentration; a trace element; an inorganic salt (e.g., sodium bicarbonate); or any combination thereof.

3. The culture medium according to claim 1 or 2, wherein the poultry egg content is egg yolk and/or egg white;
preferably, the culture medium does not contain an antibiotic;
preferably, the egg yolk and egg white are derived from poultry eggs of the same type or different types;
preferably, in the poultry egg content, the ratio of egg yolk to egg white is 99:1 to 1:99.

4. The culture medium according to any one of claims 1 to 3, wherein the culture medium contains or does not contain a fetal bovine serum.

5. Use of the culture medium according to any one of claims 1 to 4 in cultivating mammalian cell; preferably, the cultivating is adherent cultivating, suspension cultivating or immobilization cultivating.

6. The use according to claim 5, wherein the culture medium is mixed with another culture medium that is applicable to cultivating mammalian cell, including but not limited to mixing with RPMI-1640, DMEM, MEM, M199, F-10, F-12, DMEM/F-12 1:1 or McCoy's 5A medium.

7. The use according to claim 5 or 6, wherein the mammalian cells have one or more of the following characteristics:
(1) the mammalian cells are derived from a bovine, equine, swine, canine, feline, rodent or primate;
(2) the mammalian cells are derived from a connective tissue, muscle tissue, nerve tissue or epithelial tissue;
(3) the mammalian cells are derived from heart, bladder, ovary, pancreas, breast, liver, lung, stomach, kidney, intestine, skin, bone, muscle, esophagus, trachea, male reproductive system, female reproductive system, lymph, nerve or thyroid;
(4) the mammalian cells are non-tumor cells or tumor cells;
(5) the mammalian cells are cells of single type, or a mixture of cells of multiple types.

8. A method for preparing the culture medium according to any one of claims 1 to 4, the method comprising the following steps:
Step 1: obtaining a poultry egg content in an aseptic condition;
Step 2: diluting the poultry egg content with a diluent;
optionally, the method further comprises: adding an antibiotic, phenol red, glucose, vitamin, inorganic salt, fetal bovine serum or any combination thereof to the poultry egg content.

9. A method for cultivating mammalian cells, the method comprising using the culture medium according to any one of claims 1 to 4;
preferably, the cultivating is adherent cultivating, suspension cultivating or immobilization cultivating.

10. The method according to claim 9, the method comprising: mixing the culture medium according to any one of claims 1-4 to with another medium that is applicable to cultivating mammalian cell, e.g. mixing with RPMI-1640, DMEM, MEM, M199, F-10, F-12, DMEM/F-12 1:1 or McCoy's 5A medium.
